# EUROPEAN PATENT SPECIFICATION

(11) **EP 1 773 273 B1**
(45) Date of publication and mention of the grant of the patent: **04.07.2012**
(21) Application number: 04775299.3
(22) Date of filing: 06.08.2004
(51) Int. Cl.: A61F 13/56, A61F 13/60, A61F 13/62, A61F 13/66

(54) **AN ABSORBENT ARTICLE**
SAUGFÄHIGER ARTIKEL
ÉLÉMENT ABSORBANT

(43) Date of publication of application: 18.04.2007
(73) Proprietor: SCA Hygiene Products AB, 405 03 Göteborg (SE)
(72) Inventor: WINQVIST, Pontus, S-44460 Stora Höga (SE)
(74) Representative: VALEA AB
(86) International application number: PCT/SE2004/001176
(87) International publication number: WO 2006/014127

(56) References cited:
- WO-A1-01/58402
- WO-A1-90/04374
- WO-A1-98/38956
- JP-A- 10 012 733
- US-B1- 6 307 120

## Description

### FIELD OF THE INVENTION

of subsequent claim 1.

### BACKGROUND OF THE INVENTION AND PRIOR ART

As a rule, incontinence products for adults are constituted of a liquid-impervious backsheet and a liquid-pervious topsheet and an absorbent core arranged between these two layers. The liquid-pervious layer is intended to be applied closest to the body, whereas the liquid-impervious layer constitutes the outside of the product. The absorbent core should have the capability to rapidly accommodate a relatively large liquid quantity, and the core should further be able to distribute and store the liquid.

The absorbent product has a front portion and a back portion which when in use are positioned in front of and behind, respectively, the user. The front portion and the back portion terminate in a front end region and a rear end region, respectively. In order to make it possible to wear the product securely, some kind of fastening system, having the task of securing the product in its position on a user, is arranged on the product. As a rule, such a fastening system is created by means of the front and rear end regions being provided with interacting fastening members, for example in the form of a hook and loop fastening system, such as a Velcro fastener or, alternatively, a system with adhesive tape tabs. When using a hook and loop fastening system, two zones exhibiting hooks are arranged on two tabs in the rear end region, and two corresponding, separate receiving zones, exhibiting loops for interacting with the hooks when fastening, are arranged in the front end region.

WO 98/38956 discloses an absorbent article according to prior art which exhibits an absorbent core and a breathable backsheet in the form of a laminate. A continuous receiving zone of a conventional type is arranged on the outside of the backsheet. The receiving zone overlaps a portion of the core in a front region of the front portion of the absorbent article. Furthermore, it is disclosed that the backsheet as such, being in the form of a laminate composed of two different layers, can constitute a receiving zone of a hook and loop fastening system.

The previously known products for incontinent adults, however, have a number of disadvantages. Even though there are different sizes of incontinence products, each respective size is usually optimal only for a smaller group of users, since different individuals have different body sizes. It would be far too costly to adapt the incontinence product to the bodily constitution of an individual. In addition to the fact that a less than optimal fit can make the product uncomfortable to wear, also the function of the product can be impaired if the fit is insufficient. For instance, it is an advantage if a portion of the inner, liquid-pervious topsheet, corresponding to the region around the upper edge of the absorbent core of the front portion can abut and seal tightly against the body. This has the advantage that leakage is prevented. For many users of incontinence guards, this function cannot always be achieved as a result of an insufficient fit.

When the fastening function and thereby the waist size are concerned, the product could be adapted to users of different sizes by means of arranging proportionately large receiving zones for interaction with fastening members, such as hooks or the like, in the front end region of the front portion of the product. On the other hand, however, it is desirable that each respective receiving zone, which usually consists of a material layer being provided with loops, is as small as possible, since such a material layer influences the ductility and flexibility of the product adversely, since the product becomes substantially stiffer in the region where the receiving zone is arranged. Furthermore, such a material layer usually has a much lower breathability than the remainder of the product. Consequently, large receiving zones would result in an inferior comfort and increase the risk of skin irritation because of the lower breathability. Furthermore, it is a fact that larger receiving zones lead to increased material costs which, in their turn, result in a more expensive product.

### OBJECT OF THE INVENTION AND SUMMARY OF THE INVENTION

One object of the invention is to provide an absorbent article exhibiting a receiving means which substantially reduces the above-discussed problem with such previously known articles. Accordingly, the absorbent article according to the invention is provided with a receiving means which, by comparison with the previously known products of the type in question, substantially improves the fit and flexibility of the absorbent article.

This object is achieved by means of an absorbent article according to claim 1. By means of using a flexible nonwoven laminate as a receiving means, wherein the nonwoven laminate and the absorbent core overlap each other so that the overlapping zone created in that way has an extension amounting to at least 45 cm², the receiving means can be utilised for bringing the absorbent core to abut and seal tightly against the body when in use, directly or via an inner, liquid-pervious layer, so that the function is improved, at the same time as a good ductility and fit are obtained.

By means of using a nonwoven laminate, the article according to the invention can exhibit a high degree of ductility and flexibility, as well as process engineering advantages and, furthermore, a nonwoven laminate can be produced at a proportionately low cost. Altogether, these characteristics result in the possibility of producing proportionately large receiving zones without sacrificing performance or economy.

The proportionately large overlapping zone is important for obtaining the desired comfort of an incontinence product for adults, said product being very soft and flexible in itself. It has been found that a minimum overlapping zone of the indicated size, as a rule, is required for enabling the desired comfort to be achieved. The proportionately large area of the receiving means which is required in order to obtain the overlapping zone also makes it possible to vary the positioning of the tabs, being provided with e.g. hooks, when fastening the tabs to the receiving means, which means that the waist measurement and, accordingly, the size of the product can be varied to some extent. In addition, a continuous area of the receiving means results in a more rational production of the absorbent article, since the attachment of a receiving means on the outside of the article usually can be performed with fewer process steps than the attachment of two separate receiving means.

### BRIEF DESCRIPTION OF THE DRAWINGS

A detailed description of exemplifying embodiments of the invention will follow below, with reference to the attached drawings.

In the drawings:
Fig. 1 is a perspective view of an absorbent article according to the invention;
Fig. 2 is a plan view of an absorbent article according to the invention; and
Fig. 3 is a sectional view, illustrating a nonwoven laminate for providing a receiving means of the article according to the invention.

### DETAILED DESCRIPTION OF PREFERRED EMBODIMENTS OF THE INVENTION.

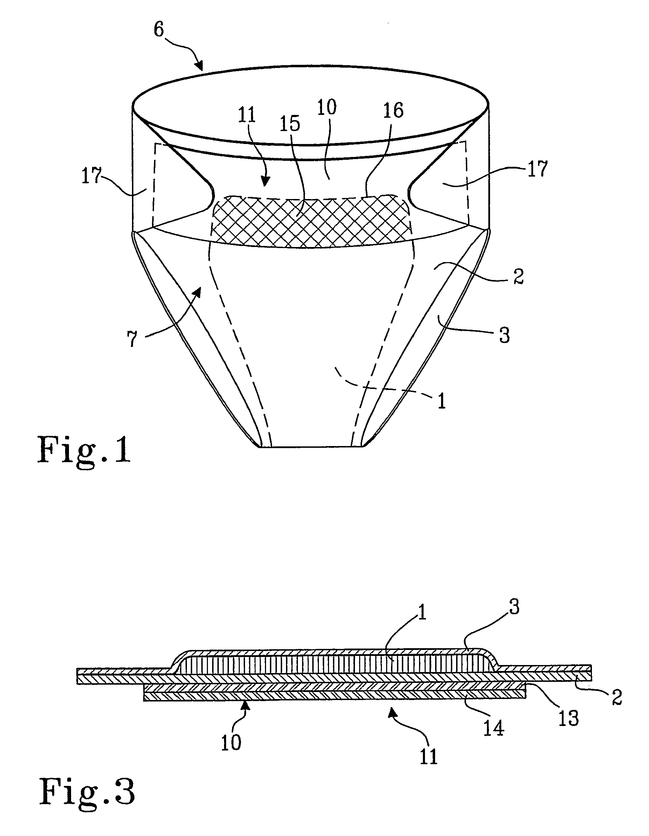

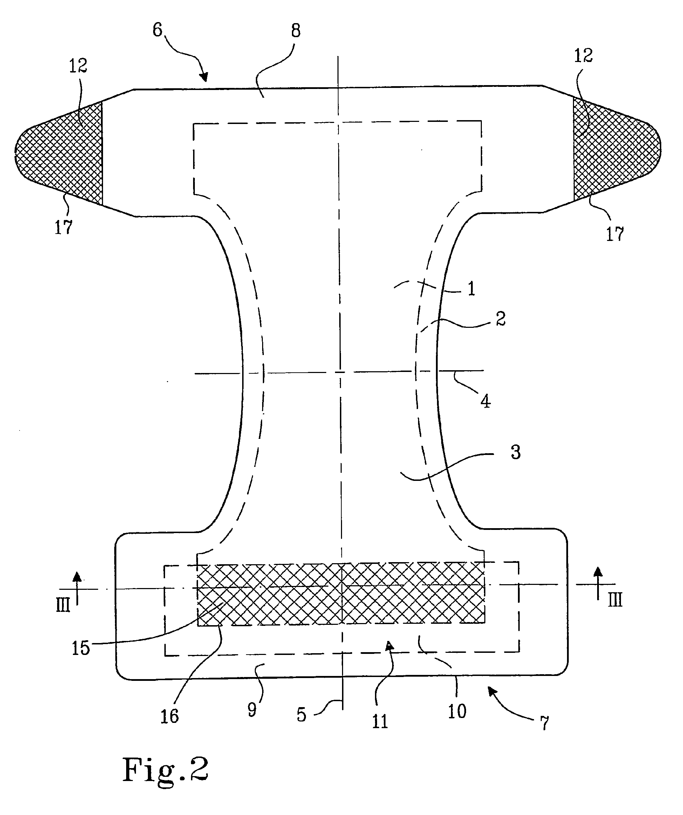

Figures 1 and 2 are representations of an absorbent article, preferably constituting an incontinence article intended for adults, which includes an absorbent core 1 being arranged between a liquid-impervious backsheet 2 and a liquid-pervious topsheet 3. The backsheet 2 constitutes the outside of the article and the topsheet 3 constitutes the inside of the article, being located closest to the body when in use. The article has a transverse 4 and a longitudinal geometrical axis 5. The transverse axis 4 divides the article into a back portion 6 and a front portion 7. The back portion 6 exhibits a rear end region 8 and the front portion 7 exhibits a front end region 9. The backsheet 2 can consist of one, two or several layers, and preferably is a breathable layer. For example, the backsheet 2 can consist of an inner layer, which is liquid-impervious and breathable and arranged closest to the core, and an outer, breathable layer which is arranged on top of it. The topsheet 3 can consist of one or several layers of liquid-pervious material. The core 1 can be made of various materials, such as cellulose pulp, woven fabrics, nonwoven fabrics, fibrous materials, foam materials, superabsorbent materials, synthetic materials, such as thermoplastic fibres, etc.

According to the invention, a receiving means 11 is arranged on the opposite side of the backsheet 2 in relation to the core 1, in order to provide a surface 10 for receiving fastening members 12 arranged on the article. The receiving means 11 and the fastenings members 12 are adapted for being joined together when applying the article onto a user. As illustrated in Figure 3, the receiving means is a nonwoven laminate 11, including at least a first, inner layer 13 and at least a second, outer layer 14, said nonwoven laminate 11 being arranged on the outside of the backsheet 2 for receiving fastening members 12 which are arranged on the article. Preferably, the fastening members 12 are arranged in the rear end region 8, and are, accordingly, intended to be joined to the nonwoven laminate 11 when applying the article onto a user. Preferably, the nonwoven laminate 11 is adapted for receiving fastening members 12 in the form of hooks in order to create a hook and loop fastening system. One way of achieving a suitable surface for receiving hooks is to make the nonwoven laminate by means of carding. Preferably, the nonwoven laminate 11 is a flexible laminate which includes said first, inner layer 13 of spunbond nonwoven and said second, outer layer 14 of carded nonwoven for providing the receiving surface 10 for the fastening members 12.

The nonwoven laminate 11 and the absorbent core 1 overlap each other so that the overlapping zone 15 created in this way (being shadowed in Figures 1 and 2 solely for illustrative purposes) has an extension amounting to at least 45 cm². Preferably, the extension of the overlapping zone 15 is at least 60 cm², and more preferably at least 75 cm², and most preferably at least 100 cm². The area of the overlapping zone is measured when the absorbent article is placed in its planar, extended condition, as illustrated in Figure 2. In the front end region 9 of the front portion 7, the core 1 exhibits a peripheral edge 16 whose main extension in a longitudinal direction is substantially parallel to the transverse geometrical axis 4. As a rule, this peripheral edge 16 of the core 1 extends substantially in parallel with the transverse geometrical axis 4 and substantially linearly across the front portion 7. It is advantageous that the overlapping zone 15 includes at least one portion of said peripheral edge 16, and preferably the overlapping zone 15 includes the major part of or substantially the entire peripheral edge 16, for the purpose of getting the topsheet 3 and/or the core 1 to seal tightly against the body of a user.

The rear end region 8 of the back portion 6 can be provided with two tabs 17. Each respective tab 7 is provided with said fastening members 12, preferably hooks, for enabling it to be attached to the nonwoven laminate 11. The surface 10 of the nonwoven laminate 11 of the outer layer 14, for example carded nonwoven, can provide loops to which loops the hooks can be fastened.

Suitably, the nonwoven laminate 11 is breathable in order to avoid a deterioration of the breathability of the article. The breathability of the nonwoven laminate 11 is preferably larger than or equal to the breathability of the backsheet 2. The breathability of the nonwoven laminate 11, expressed as a MVTR-value (mass vapour transmission rate) can be larger than or equal to 2000 g/m²/24h, preferably ≥ 4000 g/m²/24h, and most preferably ≥ 6000 g/m²/24h. The MVTR-value can be determined in accordance with the testing method ASTM E-960.

One example of a nonwoven laminate which is particularly well suited for implementing the invention is a spunbond/carded nonwoven laminate where the spunbond layer constitutes the first, inner layer 13 of the laminate and the carded layer constitutes the second, outer layer 14 of the laminate. For such a nonwoven laminate, the following layers can be used, viz. a white spunbond layer of 100 % polypropylene having a basis weight which is 30 g/m², made of fibres within the interval 2.2 to 2.5 dtex, whereas the carded layer can be a white layer of 100 % polypropylene, having a basis weight which is 30 g/m², made of 2.2 dtex fibres.

A basis weight of the inner and/or outer layer which is lower than 30 g/m² can be used, however, since the stress normally is not particularly high. Accordingly, a lower basis weight of, for example, 18 g/m² is normally acceptable. The basis weight is preferably within the interval between 15 g/m² and 80 g/m², and more preferably between 20 and 65 g/m², in order to meet the demands for both strength and softness. Basis weight measurements should be performed by means of using dry materials at a temperature of 21 °C and a humidity of 55 %.

The above-mentioned carded layer (white, 100 % polypropylene, 2.2 dtex, between 18 and 30 g/m²) can also be used as the surface layer facing outwards towards the garment, onto which surface layer a material section being provided with hook-like members (of a hook and loop fastening system), of a fastening tab which is intended to be utilised when the product has been used, can be attached in a detachable way.

The layers of the nonwoven laminate can be bonded into a laminate by means of using ultrasonics, but also other binding methods, such as gluing, heat welding etc., or combinations of said methods, can be utilised. The same binding methods can be utilised for attaching the laminate to the backsheet. For cost reasons, however, ultrasonic bonding is usually preferred. The total bonded area of the layers can be 3-20 %, and preferably of the magnitude 5-10 %, of the total area of the laminate.

It should be noted, however, that a number of different nonwoven materials can be utilised for creating the laminate according to the invention.

The nonwoven laminate suitably has a stiffness (Peak bending stiffness) which is equal to or lower that 40 gf (gram force), and preferably the stiffness is lower than 25 gf. The stiffness is measured in accordance with the modified testing method disclosed in US 5,009,653. The testing method is a modification of ASTM D 4032.82 "CIRCULAR BEND PROCEDURE".

Advantageously, the nonwoven laminate can be made liquid-pervious. As a rule, a liquid-pervious laminate requires a lower level of quality control, since larger tolerances of the material can be accepted, something which in its turn can rationalize the production and offer savings.

The liquid-pervious topsheet 3 can be made of a nonwoven material, such as spunbond, meltblown, carded, hydroentangled, wetlaid nonwoven, etc. Suitable nonwoven materials can be composed of natural fibres, such as cellulose or cotton fibres, manmade fibres, such as polyester, polyethylene, polypropylene, viscose, etc. or a mixture of natural fibres and manmade fibres. Furthermore, the topsheet material can be composed of tow-fibres, which can be bonded to each other in a bonding pattern, as disclosed in e.g. EP-A-1 035 818. Additional examples of topsheet materials are porous foams, apertured plastic films, etc. The materials being arranged as topsheet material should be soft and not cause any skin irritation and allow easy penetration of body fluid, such as urine and menstrual fluid. Furthermore, the topsheet can be different in different portions of the absorbent article.

The liquid-impervious backsheet 2, covering the core region on the side of the article facing outwards towards the clothes when in use, is made of a liquid-impervious material, such as a thin plastic film, e.g. a polyethylene or polypropylene film, a nonwoven material coated with a liquid-impervious material, a hydrophobic nonwoven material counteracting or preventing liquid from penetrating the material, or laminates of plastic films and nonwoven materials. The backsheet can be breathable at the core region in order to allow water vapour to be emitted from the absorbent core, while liquid still is prevented from passing through the layer.

Examples of breathable backsheet materials are porous films, nonwoven laminates of spunbond and meltblown layers, laminates of porous polymer films and nonwoven materials. Preferably, the backsheet is inelastic.

Films made of polyolefins filled with solid particles (e.g. a mineral such as calcium carbonate) can be mentioned as examples of backsheets of the article. In certain conditions, these films are known to form porous structures, enabling diffusion of water vapour while a liquid barrier is provided. This is a desirable characteristic of hygiene articles of different types. A considerable part of the comfort of an absorbent article is actually its ability to maintain a balance between heat generation and heat loss. Heat loss through a covering can arise from direct, dry heat loss, or from evaporation of moisture. When the latter phenomenon is concerned, the rate at which moisture is transformed into vapour by the material being used in order to form the garment or the absorbent article is generally related to the breathability of the material. The breathability is the ability to diffuse moisture/water vapour through a film or an absorbent article. In addition to this characteristic, there are many applications requiring that the material which is used in order to achieve the absorbent article is liquid-impervious. Such applications include backsheets of diapers and incontinence guards, panty liners, medical protective articles, sterile dressings, plasters, bandages, guards for intravenous and ostomic treatment, breathable construction membranes such as protective fabrics for house foundations etc.

Films which allow water vapour to pass through and are porous, but which still are intended to be impervious for liquid, are disclosed in U.S. Patent Nos. 4,626,252 and 5,073,316. As described therein, a porous film is obtained by means of mixing a polyolefin plastic, an inorganic filler and a softener; forming a film of the mixture; and uniaxial and biaxial stretching of the film. Films of this type are also disclosed in U.S. Patent No. 5,998,505 and the PCT-application with publication number WO98/05501.

It is emphasised that the invention is not restricted to the embodiments of the invention which have been described as examples, but a number of variants and modifications of the invention within the scope of the following claims will become obvious to the skilled person once the inventive idea has been disclosed.

## Claims

1. An absorbent article including an absorbent core (1) and a liquid-impervious backsheet (2), wherein a receiving means (11) is arranged on the opposite side of the backsheet (2) in relation to the core (1) in order to provide a surface (10) for receiving fastening members (12) arranged on the article, and the receiving means (11) and the fastening members (12) are adapted for being joined together when applying the article onto a user, **characterized in that** the receiving means is a flexible nonwoven laminate (11), including at least one inner (13) and one outer (14) layer, and that the nonwoven laminate (11) and the absorbent core (1) overlap each other so that the overlapping zone (15) formed **in that** way has an extension amounting to at least 45 cm².

2. Article according to claim 1, **characterized in that** the extension of the overlapping zone (15) is at least 60 cm².

3. Article according to claim 1, **characterized in that** the extension of the overlapping zone (15) is at least 75 cm².

4. Article according to claim 1, **characterized in that** the extension of the overlapping zone (15) is at least 100 cm².

5. Article according to any one of the preceding claims, **characterized in that** the inner laminate layer (13) is made of spunbond nonwoven and the outer laminate layer (14) is made of carded nonwoven.

6. Article according to any one of the preceding claims, **characterized in that** the nonwoven laminate (11) is adapted for receiving fastening members (12) in the form of hooks of a hook and loop fastening system.

7. Article according to any one of the preceding claims, **characterized in that** the article has a transverse geometrical axis (4), dividing the article into a front portion (7) and a back portion (6), and that the core (1) exhibits a peripheral edge (16) whose main extension in a longitudinal direction is substantially parallel to the transverse geometrical axis (4), wherein the overlapping zone (15) includes at least a portion of said peripheral edge (16).

8. Article according to claim 7, **characterized in that** the overlapping zone (15) includes the major part of, or substantially the entire peripheral edge (16).

9. Article according to claim 7 or 8, **characterized in that** said peripheral edge (16) is arranged in the front portion (7).

10. Article according to any one of the preceding claims, **characterized in that** the nonwoven laminate (11) is breathable.

11. Article according to claim 10, **characterized in that** the nonwoven laminate (11) has a breathability expressed as a MVTR-value which is ≥ 2000 g/m²/24h measured according to ASTM E-960.

12. Article according to claim 10, **characterized in that** the nonwoven laminate (11) has a breathability expressed as a MVTR-value which is ≥ 4000 g/m²/24h measured according to ASTM E-960.

13. Article according to claim 10, **characterized in that** the nonwoven laminate (11) has a breathability expressed as a MVTR-value which is ≥ 6000 g/m²/24h measured according to ASTM E-960.

14. Article according to any one of the preceding claims, **characterized in that** the nonwoven laminate (11) has a stiffness (measured in accordance with the testing method referred to in the description) which is lower than or equal to 40 gf, and preferably lower than 25 gf.

15. Article according to any one of the preceding claims, **characterized in that** the inner laminate layer (13) has a basis weight within the interval 15-80 g/m², and preferably within the interval 20-65 g/m².

16. Article according to any one of the preceding claims, **characterized in that** the outer laminate layer (14) has a basis weight within the interval 15-80 g/m², and preferably within the interval 20-65 g/m².

17. Article according to any one of the preceding claims, **characterized in that** it includes a liquid-pervious topsheet (3), wherein the core (1) is arranged between the liquid-impervious backsheet (2) and the liquid-pervious topsheet (3).

18. Article according to any one of the preceding claims, **characterized in that** the article constitutes an incontinence guard for an adult.

19. Article according to any one of the preceding claims, **characterized in that** the breathability of the nonwoven laminate (11) is larger than or equal to the breathability of the backsheet (2).

20. Article according to any one of the preceding claims, **characterized in that** the nonwoven laminate (11) is liquid-pervious.

## Patentansprüche

1. Saugfähiger Artikel mit einem saugfähigen Kern (1) und einer flüssigkeitsundurchlässigen Rücklage (2), wobei ein Aufnahmemittel (11) auf der gegenüberliegenden Seite der Rücklage (2) in Beziehung zu dem Kern (1) angeordnet ist, um eine Fläche (10) zum Aufnehmen von Befestigungselementen (12) bereitzustellen, die auf dem Artikel angeordnet sind, und das Aufnahmemittel (11) und die Befestigungselemente (12) angepasst sind, miteinander verbunden zu werden, wenn der Artikel auf einen Benutzer aufgebracht wird, **dadurch gekennzeichnet, dass** das Aufnahmemittel ein flexibles nicht gewebtes Laminat (11) ist, dass mindestens eine innere (13) und eine äußere (14) Schicht aufweist, und das sich das nicht gewebte Laminat (11) und der saugfähige Kern (1) so überlappen, dass die auf diese Weise ausgebildete Überlappungszone (15) eine Erweiterung aufweist, die mindestens 45 cm² beträgt.

2. Artikel nach Anspruch 1, **dadurch gekennzeichnet, dass** die Erweiterung der Überlappungszone (15) mindestens 60 cm² beträgt.

3. Artikel nach Anspruch 1, **dadurch gekennzeichnet, dass** die Erweiterung der Überlappungszone (15) mindestens 75 cm² beträgt.

4. Artikel nach Anspruch 1, **dadurch gekennzeichnet, dass** die Erweiterung der Überlappungszone (15) mindestens 100 cm² beträgt.

5. Artikel nach einem der vorstehenden Ansprüche, **dadurch gekennzeichnet, dass** die innere Laminatschicht (13) aus einem Spinnvlies hergestellt ist und die äußere Laminatschicht (14) aus einem kardierten Vlies hergestellt ist.

6. Artikel nach einem der vorstehenden Ansprüche, **dadurch gekennzeichnet, dass** das nicht gewebte Laminat (11) zum Aufnehmen von Befestigungselementen (12) in Form von Haken eines Haken- und Schlaufenbefestigungssystems angepasst ist.

7. Artikel nach einem der vorstehenden Ansprüche, **dadurch gekennzeichnet, dass** der Artikel eine querlaufende Geometrieachse (4) aufweist, die den Artikel in einen vorderen Abschnitt (7) und einen hinteren Abschnitts (6) teilt, und dass der Kern (1) eine Umfangskante (16) aufweist, deren Haupterweiterung in einer Längsrichtung im Wesentlichen parallel zu der querlaufenden Geometrieachse (4) ist, wobei die Überlappungszone (15) mindestens einen Abschnitt der Umfangskante (16) einschließt.

8. Artikel nach Anspruch 7, **dadurch gekennzeichnet, dass** die Überlappungszone (15) den Hauptteil oder im Wesentlichen die gesamte Umfangskante (16) einschließt.

9. Artikel nach Anspruch 7 oder 8, **dadurch gekennzeichnet, dass** die Umfangskante (16) in dem vorderen Abschnitt (7) angeordnet ist.

10. Artikel nach einem der vorstehenden Ansprüche, **dadurch gekennzeichnet, dass** das nicht gewebte Laminat (11) atmungsaktiv ist.

11. Artikel nach Anspruch 10, **dadurch gekennzeichnet, dass** das nicht gewebte Laminat (11) eine als MVTR-Wert ausgedrückte Atmungsaktivität aufweist, die gemessen nach ASTM E-960 2000 g/m²/24h ist.

12. Artikel nach Anspruch 10, **dadurch gekennzeichnet, dass** das nicht gewebte Laminat (11) eine als MVTR-Wert ausgedrückte Atmungsaktivität aufweist, die gemessen nach ASTM E-960 ≥ 4000 g/m²/24h ist.

13. Artikel nach Anspruch zehn, **dadurch gekennzeichnet, dass** das nicht gewebte Laminat (11) eine als MVTR-Wert ausgedrückte Atmungsaktivität aufweist, die gemessen nach ASTM E-960 ≥ 6000 g/m²/24h ist.

14. Artikel nach einem der vorstehenden Ansprüche, **dadurch gekennzeichnet, dass** das nicht gewebte Laminat (11) eine Steifigkeit aufweist (gemessen in Übereinstimmung mit dem in der Beschreibung genannten Testverfahren), die kleiner als oder gleich 40 gf und bevorzugt kleiner als 25 gf ist.

15. Artikel nach einem der vorstehenden Ansprüche, **dadurch gekennzeichnet, dass** die innere Laminatschicht (13) ein Flächengewicht in dem Intervall 15-80 g/m² und bevorzugt in dem Intervall 20-65 g/m² aufweist.

16. Artikel nach einem der vorstehenden Ansprüche, **dadurch gekennzeichnet, dass** die äußere Laminatschicht (14) ein Flächengewicht in dem Intervall 15-80 g/m² und bevorzugt in dem Intervall 20-65 g/m² aufweist.

17. Artikel nach einem der vorstehenden Ansprüche, **dadurch gekennzeichnet, dass** er eine flüssigkeitsdurchlässige Oberlage (3) aufweist, wobei der Kern (1) zwischen der flüssigkeitsundurchlässigen Rücklage (2) und der flüssigkeitsdurchlässigen Oberlage (3) angeordnet ist.

18. Artikel nach einem der vorstehenden Ansprüche, **dadurch gekennzeichnet, dass** der Artikel einen Inkontinenzschutz für einen Erwachsenen bildet.

19. Artikel nach einem der vorstehenden Ansprüche, **dadurch gekennzeichnet, dass** die Atmungsaktivität des nicht gewebten Laminats (11) größer als oder gleich der Atmungsaktivität der Rücklage (2) ist.

20. Artikel nach einem der vorstehenden Ansprüche, **dadurch gekennzeichnet, dass** das nicht gewebte Laminat (11) flüssigkeitsdurchlässig ist.

## Revendications

1. Article absorbant comprenant un coeur absorbant (1) et une feuille de dos (2) imperméable aux liquides, dans lequel un moyen de réception (11) est disposé sur la face de la feuille de dos (2) opposée par rapport au coeur (1), afin de fournir une surface (10) destinée à recevoir des éléments de fixation (12) disposés sur l'article, le moyen de réception (11) et les éléments de fixation (12) étant adaptés pour être joints ensemble lorsque l'article est appliqué sur un utilisateur, **caractérisé en ce que** le moyen de réception est un stratifié flexible (11) en non-tissé, comprenant au moins une couche interne (13) et une couche externe (14), et **en ce que** le stratifié (11) en non-tissé et le coeur absorbant (1) se recouvrent l'un l'autre de telle sorte que la zone de recouvrement (15) ainsi formée s'étend sur au moins 45 cm².

2. Article conforme à la revendication 1, **caractérisé en ce que** la zone de recouvrement (15) s'étend sur au moins 60 cm².

3. Article conforme à la revendication 1, **caractérisé en ce que** la zone de recouvrement (15) s'étend sur au moins 75 cm².

4. Article conforme à la revendication 1, **caractérisé en ce que** la zone de recouvrement (15) s'étend sur au moins 100 cm².

5. Article conforme à l'une des revendications précédentes, **caractérisé en ce que** la couche interne (13) du stratifié est faite d'un non-tissé filé-lié et la couche externe (14) du stratifié est faite d'un non-tissé cardé.

6. Article conforme à l'une des revendications précédentes, **caractérisé en ce que** le stratifié (11) en non-tissé est adapté pour recevoir des éléments de fixation (12) qui ont la forme des crochets d'un système de fixation à boucles et crochets.

7. Article conforme à l'une des revendications précédentes, **caractérisé en ce que** l'article présente un axe géométrique transversal (4) qui divise l'article en une partie avant (7) et une partie arrière (6), et **en ce que** le coeur (1) présente un bord périphérique (16) dont l'extension principale dans une direction longitudinale est pratiquement parallèle à l'axe géométrique transversal (4), la zone de recouvrement (15) incluant au moins une partie dudit bord périphérique (16).

8. Article conforme à la revendication 7, **caractérisé en ce que** la zone de recouvrement (15) inclut la majeure partie du bord périphérique (16), ou pratiquement sa totalité.

9. Article conforme à la revendication 7 ou 8, **caractérisé en ce que** ledit bord périphérique (16) est disposé dans la partie avant (7).

10. Article conforme à l'une des revendications précédentes, **caractérisé en ce que** le stratifié (11) en non-tissé est perméable à la vapeur d'eau.

11. Article conforme à la revendication 10, **caractérisé en ce que** le stratifié (11) en non-tissé présente une perméabilité à l'humidité sous forme vapeur qui, exprimée en termes de vitesse de transmission d'humidité à l'état vapeur et mesurée selon la norme ASTM E-960, est supérieure ou égale à 2000 g/m².24h.

12. Article conforme à la revendication 10, **caractérisé en ce que** le stratifié (11) en non-tissé présente une perméabilité à l'humidité sous forme vapeur qui, exprimée en termes de vitesse de transmission d'humidité à l'état vapeur et mesurée selon la norme ASTM E-960, est supérieure ou égale à 4000 g/m².24h.

13. Article conforme à la revendication 10, **caractérisé en ce que** le stratifié (11) en non-tissé présente une perméabilité à l'humidité sous forme vapeur qui, exprimée en termes de vitesse de transmission d'humidité à l'état vapeur et mesurée selon la norme ASTM E-960, est supérieure ou égale à 6000 g/m².24h.

14. Article conforme à l'une des revendications précédentes, **caractérisé en ce que** le stratifié (11) en non-tissé présente une rigidité qui, mesurée selon le protocole d'essai indiqué dans la description, est inférieure ou égale à 40 grammes-force, et de préférence inférieure à 25 grammes-force.

15. Article conforme à l'une des revendications précédentes, **caractérisé en ce que** la couche interne (13) du stratifié présente un poids surfacique situé dans la gamme allant de 15 à 80 g/m², et de préférence, dans la gamme allant de 20 à 65 g/m².

16. Article conforme à l'une des revendications précédentes, **caractérisé en ce que** la couche externe (14) du stratifié présente un poids surfacique situé dans la gamme allant de 15 à 80 g/m², et de préférence, dans la gamme allant de 20 à 65 g/m².

17. Article conforme à l'une des revendications précédentes, **caractérisé en ce qu'**il comprend une feuille de dessus (3) perméable aux liquides, le coeur (1) étant disposé entre la feuille de dos (2) imperméable aux liquides et la feuille de dessus (3) perméable aux liquides.

18. Article conforme à l'une des revendications précédentes, **caractérisé en ce que** l'article est un article de protection contre l'incontinence pour adulte.

19. Article conforme à l'une des revendications précédentes, **caractérisé en ce que** la perméabilité à l'humidité sous forme vapeur du stratifié (11) en non-tissé est supérieure ou égale à la perméabilité à l'humidité sous forme vapeur de la feuille de dos (2).

20. Article conforme à l'une des revendications précédentes, **caractérisé en ce que** le stratifié (11) en non-tissé est perméable aux liquides.
